# EUROPEAN PATENT APPLICATION

(11) **EP 1 813 280 A1**
(43) Date of publication of application: **01.08.2007**
(21) Application number: 07001607.6
(22) Date of filing: 25.01.2007
(51) Int. Cl.: A61K 36/16

(54) **Compositions comprising Ginko BiloBa derivatives for the treatment of asthmatic and allergic conditions**

(30) Priority: 27.01.2006 IT MI20060136
(71) Applicant: Pharmextracta S.r.l., 29010 Pontenure PC (IT)
(72) Inventor: Di Pierro, Francesco, 20090 Trezzano Sul Navigilo (MI) (IT)
(74) Representative: Minoja, Fabrizio

(57) **Abstract**

The present invention relates to compositions containing fractions deriving from *Ginkgo biloba,* useful for the treatment of asthmatic and allergic conditions.

## Description

### ABSTRACT OF THE INVENTION

The present invention relates to compositions containing fractions deriving from *Ginkgo biloba* useful for the treatment of asthmatic and allergic conditions.

### INTRODUCTION

Asthma can be defined, according to most modern conceptions, an "inflammatory syndrome characterized by recurrent episodes of airway obstruction, which resolve spontaneously or as a result of pharmacological intervention".

Asthma is a quite common condition, affecting in fact 5% of population (this figure being referred to Western industrialized Countries). In principle, this disorder can appear at any age, although in most cases the first attack occurs before 25 years of age and symptomatology tends to disappear in the elderly due to the decreased immune response.

Different bioactive mediators are involved in asthma:
1) acetylcholine secreted by intrapulmonary motoneurons, through its ability to interact with muscarinic receptors of the M3 type;
2) histamine released by mast-cells;
3) kinines, leukotrienes and PAF released by pro-inflammatory cells;
4) neuropeptides released by terminal dendrites of sensory neurons.

Following the release of said mediators, smooth muscles of the bronchoalveolar tract contract causing bronchoconstriction. This is followed by the real "asthma attack" in which the subject suffers from shortness of breath, cough and, optionally, appearance of the characteristic wheeze due the constriction of the airways.

Asthma being a disease with chronicity characteristics, chronic treatment is required. This mainly comprises the use of bronchodilators (beta-adrenergic agents; xanthine derivatives), anti-inflammatories (systemic or inhalatory corticosteroids) and receptor antagonists (antihistamines, and the like).

Platelet-aggregating factor (PAF) proved to be involved in asthmatic conditions. This is released from the pro-inflammatory cells involved in the asthmatic episode, by the action of some acetyl-transferases. It has in fact been proved that PAF induces bronchoalveolar hyper-responsiveness and inflammation on the respiratory tract.

Efforts to develop a product containing standardized extracts effective in preventing and combating the asthmatic syndrome have been carried out, taking into consideration the etio-pathogenetic knowledge of the disorder and the well-documented action of some substances (the role of inflammatory cells responsible for the release of histamine and pro-inflammatory agents; the role played by PAF in inducing airway hyper-responsiveness; the beneficial action exerted by nitric oxide; the effectiveness of derivatives able to increase cyclic nucleotide intracellular content), as well as the pharmacologically established activity of some vegetable derivatives.

*Ginkgo biloba* is a tree original from Asia; the extract obtained from its leaves is chemically characterized by the presence of two different active fractions: ginkgoflavone glycosides (amentoflavone, ginkgetin, isoginkgetin, sciadopitisin, bilobetin and 5-methoxybilobetin) (24%) and terpenoids (6%), among which are pentacyclic diterpenes. The latter are strong anti-PAF agents and proved active in combating the asthmatic syndrome.

*G. biloba* leaves as well as the pharmaceutical grade extract known as *Ginkgo biloba* bi-standardized dry extract (24% and 6%) also contain a terpene fraction (which chemically is a pentacyclic diterpene fraction substantially consisting of ginkgolides A, B, C, J, M and bilobalide) characterized by strong PAF-antagonistic action. As already mentioned, PAF is one of the major mediators of both local and systemic responses of allergic and asthmatic type.

A number of pharmacological studies have in fact proved that the mixture of said diterpenes prevents bronchoconstriction and/or reduces it by 90% in the Guinea pig immunized against ovoalbumin or in which shock had been induced by histamine inoculation.

Ginkgolide B is the most active diterpene, and proved effective in the therapy of asthma as shown in studies carried out on allergic and/or asthmatic subjects during bronchoconstriction crisis. Finally, the mixture of said diterpenes prevents the release of histamine from pro-inflammatory cells derived from asthmatic subjects.

### DISCLOSURE OF THE INVENTION

The present invention relates to compositions containing fractions obtained by extraction of *Ginkgo biloba* leaves.

More particularly, the present invention relates to compositions containing a *Ginkgo biloba* fraction consisting of a mixture comprising 5 non-glycosylated biflavone structures: amentoflavone, bilobetin, ginkgogetin, isoginkgogetin and sciadopitisin.

Said biflavones exert a series of activities making them extremely effective in the prevention and therapy of the asthmatic syndrome and in the prevention and treatment of allergic reactions.

According to a preferred aspect of the invention, both *Ginkgo biloba* terpenes and dimeric flavones will be present in the form of a *Ginkgo biloba* dry extract obtained as disclosed, for example, in US 5,637,302, US 5,700.468 and EP 360 556.

According to a particularly preferred aspect of the invention, both *Ginkgo biloba* terpenes and dimeric flavones will be present in the form of complexes with phospholipids, such as lecithins, phosphatidyl choline, phosphatidyl ethanolamine, phosphatidyl serine and the like, as disclosed, for example, in EP 441 279 and commercially available under the commercial name Phytosome®.

The compositions of the present invention will be administered through the oral, inhalatory or topical routes, in the form of tablets, syrups, aerosol vials and oro-pharyngeal sprays and preparations for the topical use.

Preferred forms of tablets according to the invention are multilayer tablets with different release rates, consisting of:
a) a first layer with fast (1-3 minutes) or normal (20-30 minutes) release of the active ingredient;
b) a second layer with sustained- release (4-12 hours) of the active ingredient.

The fast- or normal- release first layer a) dissolves in the gastric environment and active principles are absorbed at the intestinal level, thus providing the normal plasmatic peaks related to the actives contained in *Ginkgo biloba.*

The sustained- release second layer b) is mixed with excipients able to induce slow disintegration (not lower than 4 hours), thereby providing the retarded absorption of the active components contained therein, thus keeping effective, even plasma levels of active principles for a prolonged time.

Therefore, tanks to the multi-layer tablets of the present invention, an immediate therapeutical and local effect as well as a protracted one (blood and peripheral tissues) are attained through a single daily administration.

According to the invention, the multi-layer tablets will contain the *Ginkgo biloba* fractions indicated above in sufficient amounts to provide therapeutical levels of active principles for a time of approx. 8-12 hours.

According to the invention, the two layers of the tablet will have different contents in *Ginkgo biloba* active principles.

According to a preferred aspect of the invention, the oral compositions will contain the active principles within the following concentration ranges:
- *Ginkgo biloba* terpenes: 1 to 100 mg;
- *Ginkgo biloba* dimeric flavones: 10 to 200 mg.

According to a preferred aspect of the invention, the double-layer tablets will contain:
i) normal release layer: 5-20 mg of *Ginkgo biloba* terpenes and 10 - 40 mg of *Ginkgo biloba* dimeric flavones;
ii) sustained- release layer: 10-40 mg of *Ginkgo biloba* terpenes and 20 - 50 mg of *Ginkgo biloba* dimeric flavones.

According to a particularly preferred aspect of the invention, the double-layer tablets will contain:
i) normal release layer: 10 mg of *Ginkgo biloba* terpenes and 20 mg of *Ginkgo biloba* dimeric flavones;
ii) sustained- release layer: 20 mg of *Ginkgo biloba* terpenes and

40 mg of *Ginkgo biloba* dimeric flavones.

The layers which constitute the double-layer tablets will further contain conventional excipients, suitable to the release intended for each of them.

Typically, the first layer with immediate or normal release may contain conventional excipients, for example ligands, such as polyvinylpyrrolidone, carboxymethylcellulose, microcrystalline cellulose, lactose, saccharose, mannitol, gum arabic, pectin, gelatin and the like; disintegrants, such as sodium croscarmellose, starch, sorbitol, sodium starch glycolate, alginates, polyvinylpyrrolidone and the like; lubricants, such as talc, magnesium stearate, stearic acid, silica gel and the like; diluents and glidants, such as calcium phosphate, silicon dioxide, starch, talc and the like; coating agents, such as sodium carboxymethylcellulose, waxes, gelatin, shellac, titanium dioxide and the like; flavours and sweetening agents, such as potassium acesulfame and the like.

Typically, the second layer with sustained- release may contain conventional excipients, e.g. retarding agents, such as ethylcellulose, methylcellulose, polyvinyl acetate, methacrylic acid esters, cellulose acetate, fatty alcohols, fatty acids glyceric esters, paraffin, natural gums and hydrogenated vegetable oils, in addition to the conventional excipients mentioned above.

The multi-layer tablets of the invention will be prepared according to conventional techniques in pharmaceutical technology, for example by wet-granulation and subsequent drying, or by dry-granulation, or through compression.

The compositions of the present invention may further contain other active components, with complementary or anyway useful action for the treatment of asthma and allergic forms, such as active components of natural and/or synthetic origin having antiseptic, anti-inflammatory and/or immunomodulating activities, for example willow, echinacea, aloe, propolis, thyme, boswellia derivatives, and the like.

According to a further aspect of the invention, the compositions for the topical use will contain dosages ranging from 0.01 to 10% of *Ginkgo biloba* fraction, and they will be used for the prevention and the treatment of local allergic reactions, sun or contact erythema.

Said compositions for the topical use may further contain other active components with complementary or anyway useful action, such as active components of natural and/or synthetic origin having anti-inflammatory, analgesic, anti-itching and anti-redness activities, for example ruscus, glycyrrhiza, zanthoxylum derivatives, and the like.

According to a preferred aspect, the topical compositions of the invention will be formulated in the form of sterile cosmetic disposable gauzes for the hygiene of mucous membranes (for example the eye conjunctiva) affected with local allergic manifestations.

The compositions of the present invention may be suitably formulated for the administration through the oral, inhalatory and topical routes and will be prepared according to conventional methods well known in the pharmaceutical technique, as those described in "Remington's Pharmaceutical Handbook", Mack Publishing Co., N.Y., USA, using excipients, diluents, filler, anti-agglomerants acceptable for the intended use. Examples of preferred formulations according to the invention are tablets, multi-layer tablets, granulates, capsules, syrups, and inhalatory formulations, such as vials for aerosol and/or oro-pharyngeal sprays and topical formulations such as creams, gel, ointments, emulsions and impregnated gauzes.

The following examples report formulations according to the present invention.

### Example 1 - Gastroprotected filmed tablets

| | |
|---|---|
| *Ginkgo* terpenes | 30 mg |
| *Ginkgo* dimeric flavones | 30 mg |
| Microcel | 50 mg |
| Dicafos | 50 mg |
| Vegetable magnesium stearate | 20 mg |
| PVP CL | 20 mg |
| Silicon dioxide | 10 mg |
| Schellac | 20 mg |

### Example 2 - Double-layer tablets (normal release/sustained- release)

| | |
|---|---|
| *Ginkgo* terpenes | 15 mg |
| *Ginkgo* dimeric flavones | 15 mg |
| Microcel | 50 mg |
| Dicafos | 50 mg |
| Vegetable magnesium stearate | 20 mg |
| PVP CL | 20 mg |
| Silicon dioxide | 10 mg |
| *Ginkgo* terpenes | 30 mg |
| *Ginkgo* dimeric flavones | 30 mg |
| Microcel | 50 mg |
| Dicafos | 50 mg |
| Vegetable magnesium stearate | 20 mg |
| PVP CL | 20 mg |
| Silicon dioxide | 10 mg |
| Metholose | 50 mg |

### Example 3 - Capsules size "0"

| | |
|---|---|
| *Ginkgo* terpenes | 30 mg |
| *Ginkgo* dimers | 30 mg |
| Talc | 10 mg |
| Microcel 101 | 100 mg |

### Example 4 - Sachets for dissolution in water (100-150 ml)

| | |
|---|---|
| *Ginkgo* terpenes | 30 mg |
| *Ginkgo* dimers | 30 mg |
| Fructose | 1000 mg |
| Methocel E5 | 20 mg |
| Aerosol | 50 mg |
| Sweetening agent Acesulfame K | 10 mg |
| E110 | 2 mg |
| Citrus flavour | 150 mg |

### Example 5 - Cream

| | |
|---|---|
| *Ginkgo* terpenes | 1% |
| *Ginkgo* dimers | 1% |
| Lauric acid triglycerides | 0.1% |
| Polysorbate 20 | 0.2% |
| Sorbitan stearate | 0.2% |
| Propylene glycol | 20% |
| Sorbitol | 1% |
| Methylparaben | 2% |
| Propylparaben | 2% |
| Butylparaben | 2% |
| Bha | 1% |
| Water | q.s. |

## Claims

1. Compositions for the treatment of asthmatic and allergic conditions, containing *Ginkgo biloba* terpenes and dimeric flavones.

2. Compositions as claimed in claim 1, containing the active components within the following ranges:
• *Ginkgo biloba* terpenes: 1 to 100 mg;
• *Ginkgo biloba* dimeric flavones: 10 to 200 mg.

3. Compositions as claimed in claims 1 - 2, wherein both terpenes and dimeric flavones from *Ginkgo biloba* are present in the form of *Ginkgo biloba* dry extract.

4. Compositions according to the above claims, both terpenes and dimeric flavones from *Ginkgo biloba* are present in the form of complexes with phospholipids, such as lecithins, phosphatidyl choline, phosphatidyl ethanolamine, phosphatidyl serine and the like.

5. Compositions according to the above claims, for the oral administration.

6. Compositions as claimed in claim 5, in the form of multi-layer tablets, comprising:
i) a fast- or normal- release layer, containing: 5 - 20 mg of *Ginkgo biloba* terpenes and 10 - 40 mg of *Ginkgo biloba* dimeric flavones;
ii) a sustained- release layer, containing: 10 - 40 mg of *Ginkgo biloba* terpenes and 20 - 50 mg of *Ginkgo biloba* dimeric flavones.

7. Compositions as claimed in claim 6, comprising:
i) a fast- or normal- release layer, containing 10 mg of *Ginkgo biloba* terpenes and 20 mg of *Ginkgo biloba* dimeric flavones;
ii) a sustained- release layer, containing 20 mg of *Ginkgo biloba* terpenes and 40 mg of *Ginkgo biloba* dimeric flavones.

8. Compositions as claimed in claims 1 - 4, for the inhalatory administration.

9. Compositions as claimed in claim 8, in the form of spray and aerosol.

10. Compositions as claimed in claims 1 - 4, for the topical administration.

11. Compositions as claimed in claim 10, in the form of creams, gel, ointments, emulsions and impregnated gauzes.

12. The use of *Ginkgo biloba* terpenes and dimeric flavones for the preparation of a medicament for the treatment of asthmatic and allergic conditions.
